# EUROPEAN PATENT APPLICATION

(11) **EP 1 970 366 A1**
(43) Date of publication of application: **17.09.2008**
(21) Application number: 06834346.6
(22) Date of filing: 08.12.2006
(51) Int. Cl.: C07C 45/85, A23L 1/30, C07C 49/743, C08B 37/16, A61K 9/20, A61K 9/48, A61K 31/122, A61K 36/60, A61K 47/40

(54) **ISOHUMULONE COMPOUND CLATHRATE AND COMPOSITION CONTAINING THE SAME**

(30) Priority: 08.12.2005 JP 2005355268
(71) Applicant: Kirin Holdings Kabushiki Kaisha, Tokyo 104--8288 (JP)
(72) Inventor: TATEWAKI, Hisanori, Takasaki-shi, Gunma 370-1295 (JP); ISHIWAKI, Naomu, Takasaki-shi, Gunma 370-1295 (JP); AWAYAMA, Hitoshi, Takasaki-shi, Gunma 370-1295 (JP)
(74) Representative: HOFFMANN EITLE
(86) International application number: PCT/JP2006/324591
(87) International publication number: WO 2007/066773

(57) **Abstract**

An object of the present invention is to provide an inclusion compound in which isohumulones are included in γ-cyclodextrin (CD). According to the present invention, there is provided an inclusion compound of isohumulones which is obtainable by mixing isohumulones, γ-cyclodextrin (γCD) and an aqueous solvent in a ratio of isohumulones : γCD : aqueous solvent = 1 : 5 to 25 : 20 to 112.5, where γCD : aqueous solvent = 1 : 4 to 9.

## Description

### Field of the Invention

The present invention relates to an inclusion compound of isohumulones in which isohumulones are included in γ-cyclodextrin (CD), and a composition containing the same, as well as a composition obtainable by mixing isohumulones and γCD in a determined ratio.

### Background Art

The isohumulones, such as isohumulone, isocohumulone and isoadohumulone, are derived by isomerizing humulones contained in hop cones, such as humulone, cohumulone and adohumulone, and are known as primary ingredients for bitter taste of beer and low-malt beer. In addition to bitter tasting, a hop plays various roles, such as addition of characteristic flavors, preservation, clarification and improvement of head retention in brewing of beer or low-malt beer. The hop (*Humulus lupulus L*.) belongs to *Cannabaceae,* and is a dioecious and perennial bine, probably originated from the area between West Asia and Central Europe. The hop grows naturally in a comparatively cold district and commercially cultivated for marketing in Germany, USA, Czech, China, *etc.* There are many agricultural species, and their typical examples are: Hersbrucker, Tettnanger, Saaz, Hallertau, Northern Brewer, Brewers Gold, Fuggle and Cluster.

It has been confirmed that the isohumulones have, in addition to the role in brewing of beer or low-malt beer, various beneficial physiological effects. Examples include: a cariostatic effect (Japanese Patent Laid-Open Publication No. 211219/1988), anti-osteoporosis effect (Japanese Patent Laid-Open Publication No. 330594/1995), growth inhibitory effect on *Helicobacter pylori* (Japanese Patent Laid-Open Publication No. 25247/1998), aldose reductase inhibition effect (Japanese Patent Laid-Open Publication No. 226640/2003), and improving effect on blood glucose level (Japanese Patent Laid-Open Publication No. 224795/2004). With such background it has been expected to apply the isohumulones to a health oriented food and drink, called as a health food, a function food, etc.

However, as mentioned above, the isohumulones have a characteristic bitter taste. Their content in beer or low-malt beer is around 20 ppm or lower, and the content beyond 30 ppm causes, with certain differences among individuals, slight discomfort, and beyond 50 ppm strong discomfort. However, in order to enjoy the beneficial physiological effects by eating or drinking, the amount causing such discomfort is required. Further, the isohumulones have drawbacks, such as tendency to degrade during storage, or oily properties unsuitable for food processing. Consequently, a food and drink containing high level of the isohumulones has been hardly realized.

A low molecular weight physiologically active substance originated from a plant has in general a bitter or astringent taste, and several techniques have been proposed to mitigate it. A technique using cyclodextrin (CD) has been also reported.

CD is a generic name for cyclic oligosaccharides that are obtained by making a cyclodextrin glucanotransferase (CGTase) act on starches, and have a cyclic chain structure formed by glucose molecules bonded by α-1,4-linkages. Since the second half of the 19^{th} century its existence in nature has been known, and at the beginning of the 20^{th} century Shardinger discovered a method of separation and purification of it, and its cyclic structure. In 1970s a CGTase originated from bacteria was discovered, which has accelerated the development of a production technology of crystalline CD. Currently CDs with 6, 7, and 8 polymerized glucose units are available for practical use, and named as αCD, βCD and γCD respectively.

Stereoscopically CD has a structure of a bottomless bucket, and while the outer surface of the cylinder is hydrophilic, the inner surface is characteristically hydrophobic. Due to the above nature, CD tends to include a specific organic compound as a "guest molecule" in the cavity, and such a phenomenon is called inclusion (clathration). They say a taste or solubility of a guest molecule can be modified by utilization of the inclusion effect. As for a mechanism of the inclusion, however, little concrete study has been made about the specific congruency between CD and a guest molecule. Such limited knowledge includes an opinion that the molecular weight suitable for a guest molecule is about 120 to 350 (Spec Chem, vol. 7, No. 6, p. 374 (1987)).

As the use of CD, an example is disclosed in Japanese Patent Laid-Open Publication No. 146271/1983 (Patent Document 1), in which 0.3% of a CD formulation containing 10% of γCD is added to commercial draft beer to mitigate a bitter taste. However, inclusion of isohumulones is not confirmed and a noticeable mitigating effect on the bitter taste has been hardly obtained.

A food and drink with a medicinal plant included by CD is disclosed in Japanese Patent Laid-Open Publication No. 305116/2004 (Patent Document 2), and a hop is exemplified as one of the medicinal plants. However, there is no description about an effect on isomerized hop extracts, or even about an effect on hop extracts.

As bitter taste mitigating techniques other than the technique with CD, the use of phosphatidic acid (Benecort BMI^{TN}, Kao Corporation), L-ornithine (Food Science Journal, no. 317, p. 56, 2004) are exemplified, but none of them have shown satisfactory results in mitigation of the bitter taste of isohumulones.
Patent Document 1: Japanese Patent Laid-Open Publication No.146271/1983
Patent Document 2: Japanese Patent Laid-Open Publication No.305116/2004

### Summary of the Invention

An object of the present invention is to provide isohumulones included in γCD and a composition containing the same, as well as a composition containing isohumulones with a substantially mitigated bitter taste characteristic of the isohumulones.

### First embodiment

The present inventors have found that an inclusion compound of isohumulones can be very efficiently prepared by mixing isohumulones, γCD and water in a ratio of isohumulones : γCD : water = 1 : 5 to 25 : 20 to 112.5, where γCD : water = 1 : 4 to 9 (Example 2(1)).

Therefore, according to the first embodiment of the present invention, there is provided an inclusion compound of isohumulones which is obtainable by mixing isohumulones, γCD and an aqueous solvent in a ratio of isohumulones : γCD : aqueous solvent = 1 : 5 to 25 : 20 to 112.5, where γCD : aqueous solvent = 1 : 4 to 9.

Further, according to the first embodiment of the present invention, there is also provided a composition comprising an inclusion compound of isohumulones, which is obtainable by mixing isohumulones, γCD and an aqueous solvent in a ratio of isohumulones : γCD : aqueous solvent = 1 : 5 to 25 : 20 to 112.5, where γCD : aqueous solvent = 1 : 4 to 9.

Further, according to the first embodiment of the present invention, there is also provided a composition containing isohumulones, which is obtainable by mixing isohumulones, γCD and an aqueous solvent in a ratio of isohumulones : γCD : aqueous solvent = 1 : 5 to 25 : 20 to 112.5, where γCD : aqueous solvent = 1 : 4 to 9.

### Second embodiment

The present inventors have found that at a pH of 4.0 or below an inclusion compound of isohumulones can be efficiently prepared by mixing isohumulones, γCD and water even in a ratio of isohumulones : γCD : water = 1 : 5 to 33 : 20 to 627, where γCD : water = 1 : 4 to 19 (Example 2(2)).

Therefore, according to the second embodiment of the present invention, there is provided an inclusion compound of isohumulones which is obtainable by mixing isohumulones, γCD and an aqueous solvent at a pH of 4.0 or below in a ratio of isohumulones : γCD : aqueous solvent = 1 : 5 to 33 : 20 to 627, where γCD : aqueous solvent = 1 : 4 to 19.

Further, according to the second embodiment of the present invention, there is also provided a composition comprising an inclusion compound of isohumulones, which is obtainable by mixing isohumulones, γCD and an aqueous solvent at the pH of 4.0 or below in the ratio of isohumulones : γCD : aqueous solvent = 1 : 5 to 33 : 20 to 627, where γCD : aqueous solvent = 1 : 4 to 19.

Further, according to the second embodiment of the present invention, there is also provided a composition containing isohumulones, which is obtainable by mixing isohumulones, γCD and an aqueous solvent at a pH of 4.0 or below in a ratio of isohumulones : γCD : aqueous solvent = 1 : 5 to 33 : 20 to 627, where γCD : aqueous solvent = 1 : 4 to 19.

### Third embodiment

The present inventors have also found that at a pH of 4.5 or below an inclusion compound of isohumulones can be prepared by mixing isohumulones, γCD and an aqueous solvent even in a ratio of isohumulones : γCD : water = 1 : 5 to 272 : 20 to 1088 (Examples 2 and 5).

Therefore, according to the third embodiment of the present invention, there is provided an inclusion compound of isohumulones which is obtainable by mixing isohumulones, γCD and an aqueous solvent at a pH of 4.5 or below in a ratio of isohumulones : γCD : aqueous solvent = 1 : 5 to 272 : 20 to 1088, where γCD : aqueous solvent = 1 : 4 to 9.

Further, according to the third embodiment of the present invention, there is provided a composition comprising an inclusion compound of isohumulones, which is obtainable by mixing isohumulones, γCD and an aqueous solvent at a pH of 4.5 or below in a ratio of isohumulones : γCD : aqueous solvent = 1 : 5 to 272 : 20 to 1088, where γCD : aqueous solvent = 1 : 4 to 9.

Further, according to the third embodiment of the present invention, there is provided a composition containing isohumulones, which is obtainable by mixing isohumulones, γCD and an aqueous solvent at a pH of 4.5 or below in a ratio of isohumulone : γCD : aqueous solvent = 1 : 5 to 272 : 20 to 1088, where γCD : aqueous solvent = 1 : 4 to 9.

### Fourth embodiment

The present inventors have also found that in order to prepare a composition containing isohumulones subjected to inclusion in a low final concentration, instead of preparing directly that with target low concentration of a composition containing an inclusion compound of isohumulones, a composition prepared according to the first embodiment, the second embodiment or the third embodiment is diluted under acidic conditions to prepare a composition containing an inclusion compound of isohumulones in a target concentration (Example 5 and 6).

Therefore, according to the fourth embodiment of the present invention, there is provided a composition comprising an inclusion compound of isohumulones, which is obtainable by diluting the composition according to the first embodiment with an aqueous solvent at a pH of 4.5 or below in a ratio of isohumulones : γCD : aqueous solvent = 1 : 5 to 272 : 4000 to 20000.

According to the fourth embodiment of the present invention, there is provided a composition containing isohumulones which is obtainable by diluting the composition according to the first embodiment with an aqueous solvent at a pH of 4.5 or below in a ratio of isohumulones : γCD : aqueous solvent = 1 : 5 to 272 : 4000 to 20000.

Further, according to the fourth embodiment of the present invention, there is provided a composition comprising an inclusion compound of isohumulones, which is obtainable by diluting the composition according to the second embodiment with an aqueous solvent at a pH of 4.5 or below in a ratio of isohumulones : γCD : aqueous solvent = 1 : 5 to 272 : 4000 to 20000.

Further, according to the fourth embodiment of the present invention, there is provided a composition containing isohumulones, which is obtainable by diluting the composition according to the second embodiment with an aqueous solvent at a pH of 4.5 or below in a ratio of isohumulones : γCD : aqueous solvent = 1 : 5 to 272 : 4000 to 20000.

Further, according to the fourth embodiment of the present invention, there is provided a composition comprising an inclusion compound of isohumulones, which is obtainable by diluting the composition according to the third embodiment with an aqueous solvent at a pH of 4.5 or below in a ratio of isohumulones : γCD : aqueous solvent = 1 : 5 to 272 : 4000 to 20000.

Further, according to the fourth embodiment of the present invention, there is provided a composition containing isohumulones, which is obtainable by diluting the composition according to the third embodiment with an aqueous solvent at a pH of 4.5 or below in a ratio of isohumulones : γCD : aqueous solvent = 1 : 5 to 272 : 4000 to 20000.

### Industrial Applicability

According to the present invention, there are provided an inclusion compound of isohumulones with substantially mitigated bitter taste, a composition comprising the same, and a composition comprising isohumulones with substantially mitigated bitter taste. Although a physiological function of isohumulones has been reported, technologies to efficiently mitigate or mask the bitter taste of isohumulones have not been reported yet. Therefore, the present invention paves the way to provision of a medicine or a food containing an effective amount of isohumulones expected to exert a physiological function and with substantially mitigated bitter taste.

### Brief Description of the Drawings

Figure 1 shows measurement results of absorbance of the respective samples by a spectrophotometer.
Figure 2 shows measurement results of turbid isohumulones rates of the respective samples.
Figure 3 shows measurement results of turbid isohumulones rates of the samples (pH 9) with various mixture ratios.
Figure 4 shows measurement results of turbid isohumulones rates of the samples (pH 4) with various mixture ratios.
Figure 5 shows observed appearances of samples A and B.
Figure 6 shows measurement results of retention rates of isohumulone in samples A and B.
Figure 7 shows sample A by an electromicroscopic observation (x 3000).
Figure 8 shows sample A by an electromicroscopic observation (x 8000).
Figure 9 shows γCD by an electromicroscopic observation (x 3000).
Figure 10 shows utilization rate of isohumulones to an inclusion compound and utilization rate of γCD to an inclusion compound.
Figure 11 shows an initial potential and a potential measured after a primary washing of the respective samples by a Taste Sensing System.
Figure 12 shows storage stability of the dry powdered inclusion compound of isohumulones.
Figure 13 shows storage stability of the spray-dried inclusion compound of isohumulones, as measured by an influence of the spray-drying temperatures: A, stored hermetically immediately after spray-drying; B, exposed to 60% relative humidity at 25°C for 1 week and then stored hermetically.
Figure 14 shows storage stability of the spray-dried inclusion compound of isohumulones, as measured by an influence of the pH value of a γCD solution: A, stored hermetically immediately after spray-drying; B, exposed to 60% relative humidity at 25°C for 1 week and then stored hermetically.
Figure 15 shows storage stability of the tablets with and without a coat.

### Detailed Description of the Invention

### First embodiment

According to the first embodiment of the present invention, an inclusion compound of isohumulones, a composition comprising the same and a composition containing isohumulones with substantially mitigated bitter taste can be prepared efficiently by mixing isohumulones, γCD and an aqueous solvent in a determined ratio. Mixing can be preferably done at a pH of 9 or below.

A composition according to the first embodiment of the present invention can comprise the isohumulones included in γCD in an amount of 70% or more relative to the total amount of the isohumulones.

A change of a membrane potential upon dissolution of the composition according to the first embodiment of the present invention in an aqueous solvent can be 15% or less compared with that of a solution of the isohumulones alone.

According to a preferable embodiment of the first embodiment of the present invention, there are provided an inclusion compound of isohumulones and a composition comprising the same as well as a composition containing isohumulones, which are obtainable by mixing isohumulones, γ-cyclodextrin (γCD) and an aqueous solvent in a ratio of isohumulones : γCD : aqueous solvent = 1 : 6 to 12.5 : 54 to 112.5, where γCD : aqueous solvent = 1 : 4 to 9.

A composition with a low concentration of isohumulones and an inclusion compound of isohumulones can be prepared by diluting the composition according to the first embodiment in accordance with the conditions disclosed as the fourth embodiment while the inclusion compound of isohumulones is maintained, or the bitter taste of isohumulones remains substantially mitigated.

According to the first embodiment of the present invention, there is further provided a method for producing an inclusion compound of isohumulones or a composition comprising an inclusion compound of isohumulones, comprising the step of mixing isohumulones, γCD and an aqueous solvent in a ratio of isohumulones : γCD : aqueous solvent = 1 : 5 to 25 : 20 to 112.5, where γCD : aqueous solvent = 1 : 4 to 9.

According to the aforementioned method, the produced composition can comprise the isohumulones included in γCD in an amount of 70% or more relative to the total amount of the isohumulones.

According to the aforementioned method, a change of a membrane potential of the produced composition in an aqueous solvent can be 15% or less compared with that of a solution of the isohumulones alone.

According to the aforementioned method, isohumulones, γCD and an aqueous solvent can be preferably mixed at a pH of 9 or below.

According to a preferable embodiment of the first embodiment of the present invention, there is provided a method for producing an inclusion compound of isohumulones or a composition comprising an inclusion compound of isohumulones, comprising the step of mixing isohumulones, γ-cyclodextrin (γCD) and an aqueous solvent in a ratio of isohumulones : γCD : aqueous solvent = 1 : 6 to 12.5 : 54 to 112.5, where γCD : aqueous solvent = 1 : 4 to 9.

Concentration and separation of the inclusion compound of isohumulones from the composition produced according to the aforementioned method can be carried out by a known method. For example the composition produced according to the aforementioned method is subjected to a solid-liquid separation method such as a centrifuge to recover a solid matter, at need followed by washing with water and solid-liquid separation again, to give an inclusion compound of isohumulones as a paste-like substance. The composition and the paste-like substance obtained according to the aforementioned method can be subjected to spray-drying, freeze-drying, etc. to obtain a solid-state inclusion compound of isohumulones.

### Second embodiment

According to the second embodiment of the present invention, an inclusion compound of isohumulones, a composition comprising the same and a composition containing isohumulones with substantially mitigated bitter taste can be prepared efficiently by mixing isohumulones, γCD and an aqueous solvent at a pH of 4.0 or below in a determined ratio.

A composition according to the second embodiment of the present invention can comprise the isohumulones included in γCD in an amount of 70% or more relative to the total amount of the isohumulones.

A change of a membrane potential upon dissolution of the composition according to the second embodiment of the present invention in an aqueous solvent can be 15% or less compared with that of a solution of the isohumulones alone.

A composition with a low concentration of isohumulones and an inclusion compound of isohumulones can be prepared by diluting the composition according to the second embodiment in accordance with the conditions disclosed as the fourth embodiment while the inclusion compound of isohumulonesis maintained, or the bitter taste of isohumulones remains substantially mitigated.

According to the second embodiment of the present invention, there is further provided a method for producing an inclusion compound of isohumulones or a composition comprising an inclusion compound of isohumulones, comprising the step of mixing isohumulones, γCD and an aqueous solvent at a pH of 4.0 or below in a ratio of isohumulones : γCD : aqueous solvent = 1 : 5 to 33 : 20 to 627, where γCD : aqueous solvent = 1 : 4 to 19.

According to the aforementioned method, the produced composition can comprise the isohumulones included in γCD in an amount of 70% or more relative to the total amount of the isohumulones.

According to the aforementioned method, a change of a membrane potential of the produced composition in an aqueous solvent can be 15% or less compared with that a solution of the isohumulones alone.

Concentration and separation of an inclusion compound of isohumulones from the composition produced according to the aforementioned method can be carried out by a known method. For example the composition produced according to the aforementioned method is subjected to a solid-liquid separation method such as a centrifuge to recover a solid matter, at need followed by washing with water and solid-liquid separation again, to give an inclusion compound of isohumulones as a paste-like substance. The composition and the paste-like substance obtained according to the aforementioned method can be subjected to spray-drying, freeze-drying, etc. to obtain a solid-state inclusion compound of isohumulones.

### Third embodiment

According to the third embodiment of the present invention, an inclusion compound of isohumulones, a composition comprising the same and a composition containing isohumulones with substantially mitigated bitter taste can be prepared efficiently by mixing isohumulones, γCD and an aqueous solvent under acidic conditions in a determined ratio, even when a ratio of the aqueous solvent is increased. Mixing can be preferably done at a pH of 3.5 or below. A pH during mixing can be in a range of pH 2.0 to 4.5, or more preferably pH 2.0 to 3.5.

A composition according to the third embodiment of the present invention can comprise the isohumulones included in γCD in an amount of 70% or more relative to the total amount of the isohumulones.

A change of a membrane potential upon dissolution of the composition according to the third embodiment of the present invention in an aqueous solvent can be 15% or less compared with that of a solution of the isohumulones alone.

A composition with a low concentration of isohumulones and an inclusion compound of isohumulones can be prepared by diluting the composition according to the third embodiment in accordance with the conditions disclosed as the fourth embodiment while the inclusion compound of isohumulones is maintained, or the bitter taste of isohumulones remains substantially mitigated.

According to the third embodiment of the present invention, there is further provided a method for producing an inclusion compound of isohumulones or a composition comprising an inclusion compound of isohumulones, comprising the step of mixing isohumulones, γCD and an aqueous solvent at a pH of 4.5 or below in a ratio of isohumulones : γCD : aqueous solvent = 1 : 5 to 272 : 20 to 1088, where γCD : aqueous solvent = 1 : 4 to 9.

According to the abovementioned method, the produced composition can comprise the isohumulones included in γCD in an amount of 70% or more relative to the total amount of the isohumulones.

According to the aforementioned method, a change of a membrane potential of the produced composition in an aqueous solvent can be 15% or less compared with that of a solution of the isohumulones alone.

In the abovementioned method, isohumulones, γCD and an aqueous solvent are preferably mixed at a pH of 3.5 or below. A pH during mixing can be preferably in a range of pH 2.0 to 4.5, or more preferably pH 2.0 to 3.5.

Concentration and separation of the inclusion compound of isohumulones from the composition produced according to the aforementioned method can be carried out by a known method. For example the composition produced according to the aforementioned method is subjected to a solid-liquid separation method, such as a centrifuge to recover a solid matter, at need followed by washing with water and solid-liquid separation again, to give an inclusion compound of isohumulones as a paste-like substance. The composition and the paste-like substance obtained according to the aforementioned method can be subjected to spray-drying, freeze-drying, etc. to obtain a solid-state inclusion compound of isohumulones.

### Fourth embodiment

According to the fourth embodiment of the present invention, an inclusion compound of isohumulones can be prepared efficiently by diluting a composition according to the first embodiment, a composition according to the second embodiment or a composition according to the third embodiment of the present invention under acidic conditions in a determined ratio of isohumulones, γCD and an aqueous solvent, even when a ratio of the aqueous solvent is increased.

Namely, when a composition containing isohumulones with a low concentration of isohumulones outside the composition ratios of the first embodiment, the second embodiment and the third embodiment is sought to be obtained, the composition with the desired concentration of the inclusion compound of isohumulones can be prepared efficiently by preparing a composition having a concentration within the composition ratio of the first embodiment, the composition ratio of the second embodiment, or the composition ratio of the third embodiment, and then diluting the same with water or other aqueous solvents in a ratio range of isohumulones : γCD : aqueous solvent = 1 : 5 to 272 : 4000 to 20000. Cases where the composition of the first embodiment and the composition of the second embodiment are diluted are described separately from cases where the composition of the third embodiment is diluted.

### <Cases where the composition of the first embodiment and the composition of the second embodiment are diluted>

According to Example 6, a composition with a low concentration of isohumulones and the inclusion compound of isohumulones can be produced by diluting the composition of the first embodiment or the composition of the second embodiment with an aqueous solvent under acidic conditions while the inclusion compound of isohumulones is maintained, or the bitter taste of isohumulones remains substantially mitigated. In this case, in order to maintain the inclusion of the dissolved isohumulones, or to maintain the mitigation of the bitter taste of isohumulones, an aqueous solvent such as water or various drinks having pH 4.5 or below, preferably pH 3.5 or below, can be used for the dilution. A pH after the dilution can be in a range of 2.0 to 4.5, preferably 2.0 to 3.5. The concentration of isohumulones and the inclusion compound of isohumulones after the dilution can be preferably 20 to 200 ppm.

### <Cases where the composition of the third embodiment is diluted>

It was confirmed in Example 5 that, within a certain range, an inclusion compound of isohumulones could be obtained more efficiently by preparing the inclusion compound of isohumulones at a high concentration, and then diluting it in a target concentration, than directly preparing the same at a target concentration. Specifically, it was confirmed that the inclusion compound could be formed more easily by conducting the dilution under more acidic conditions, and that with a higher ratio of γCD to isohumulones, the inclusion compound was formed more easily. It is therefore considered that the pH is an important factor and that a slight change of pH should have a grate influence on the inclusion when preparing the composition containing the isohumulones at a higher concentration and then diluting the same to a final concentration maintaining the formed inclustion compound of isohumulones. Concerning the quantity ratio of γCD / isohumulones, an increase of a ratio of γCD to isohumulones presumably leads to an increase of the contact efficiency, and thus have a grate influence on the inclusion. In light of these findings, the efficiency of the formation of the inclusion compound is established by a determined balance of the pH value and the γCD ratio. Consequently, it was confirmed that by putting a pH after dilution as X and by fitting the results of Example 5 to the product of (6.5-X)² and (γCD amount / isohumulone amount), the relationship of (6.5-X)² x (γCD amount / isohumulone amount) > 50 is established for efficient formation of the inclusion compound. In addition, (6.5-X) was derived from the findings that the inclusion compound was hardly formed at pH 6.5, and that the formation efficiency was improved under more acidic conditions. Further, since pH is a common logarithm value, (6.5-X) was squared. Further, since it was possible to balance the pH value with the γCD ratio, (6-5-X)² were multiplied by (γCD amount / isohumulone amount).

Therefore, in a case where the composition obtained according to the third embodiment is diluted at a pH of 4.5 or below in a ratio of isohumulones : γCD : aqueous solvent = 1 : 5 to 272 : 4000 to 20000, it is preferable that the dilution should be carried out so that the pH (X) after the dilution satisfies (6.5-X)² x (γCD amount / isohumulone amount) > 50, where X ≤ 4.5.

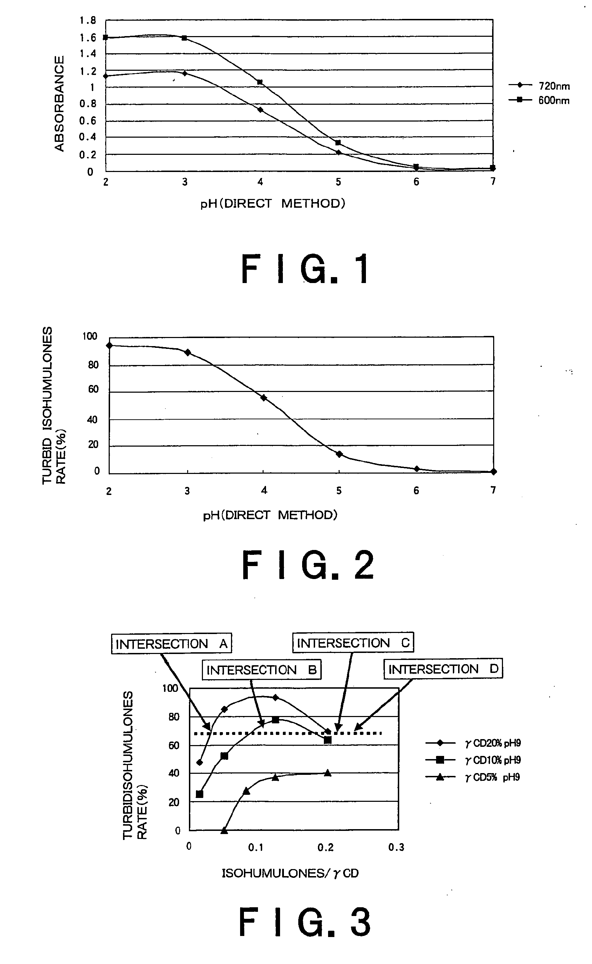

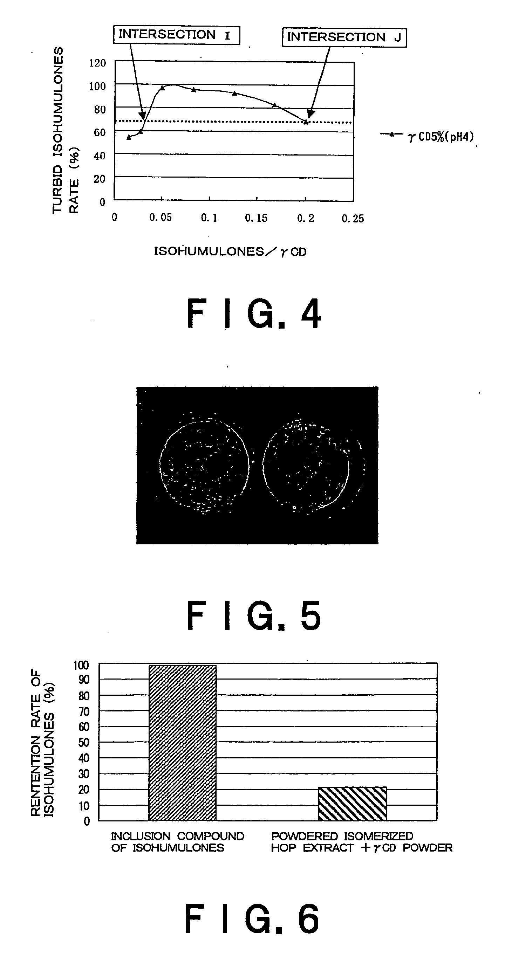

The lower the pH value is, namely, the larger the (6.5-X)² is, the higher the formation rate of a γCD inclusion compound is. Further, the higher the γCD concentration is, namely, the higher the (γCD amount / isohumulone amount) is, the higher the formation rate of a γCD inclusion compound is. Since the formation rate of a γCD inclusion compound is therefore determined by the two factors, the formation rate of a γCD inclusion compound in a γCD inclusion compound solution with a certain concentration of isohumulones can be accomplished by lowering the pH, by increasing the γCD concentration, or by lowering the pH and increasing the γCD concentration. For example, the formation rate of the γCD inclusion compound for a composition of "isohumulones : γCD : water = 1 : 12.5 : 112.5, pH 9 (γCD 10%)" is about 70%, and the formation rate of the γCD inclusion compound of the composition can be increased to 90% or higher by: (1) lowering the pH value, for example, preparing a composition of "isohumulones : γCD : water = 1 : 12.5 : 112.5, pH 4 (γCD 10%)"; (2) increasing the γCD concentration, for example preparing a composition of "isohumulones : γCD : water = 1 : 12.5 : 56.3, pH 9 (γCD 20%)"; or (3) lowering the pH value and increasing the γCD concentration, for example preparing a composition of "isohumulones : γCD : water = 1 : 12.5 : 84.4, pH 5 (γCD 15%)". As for (1), it is apparent from Figure 4 that even with isohumulones : γCD: water = 1 : 12.5 : 112.5, pH 4 (γCD 5%) the formation rate of the γCD inclusion compound is 90% or higher, and that with the 10% γCD the formation rate of the γCD inclusion compound is no less than 90%. As for (2), it is apparent from Figure 3 that an intersection of the curve of γCD 20% with the same ratio of isohumulones and γCD as isohumulones : γCD : water = 1 : 12.5 :112.5 such as intersection B indicates more than 90% of the formation rate of the γCD inclusion compound.

A pH after the dilution can be in a range of 3.5 or below. A pH after the dilution can be also in a range of 2.0 to 4.5, preferably 2.0 to 3.5.

The concentrations of the isohumulones and the inclusion compound of isohumulones in a composition after the dilution are preferably in a range of 20 to 200 ppm.

According to the fourth embodiment of the present invention, a beverage containing an inclusion compound of isohumulones can be prepared by diluting the composition according to the third embodiment with a drink. In this case, the ratio among isohumulones : γCD : aqueous solvent, and the pH value after the dilution can be set in accordance with the aforementioned conditions in order to maintain the inclusion of isohumulones after the dilution. Preferably, the dilution is carried out with a drink having pH 4.5 or below to become a ratio range of isohumulones : γCD : aqueous solvent = 1 : 5 to 272 : 4000 to 20000. The concentrations of the isohumulones and the inclusion compound of isohumulones in the drink after the dilution are preferably in a range of 20 to 200 ppm.

Further, according to the fourth embodiment of the present invention, there is provided a method for producing a composition comprising an inclusion compound of isohumulones, comprising the steps of:
(a) mixing isohumulones, γCD and an aqueous solvent in a ratio of isohumulones : γCD : aqueous solvent = 1 : 5 to 25 : 20 to 112.5, where γCD : aqueous solvent = 1 : 4 to 9; and
(b) diluting the obtained composition with an aqueous solvent at a pH of 4.5 or below in a ratio of isohumulones : γCD : aqueous solvent = 1 : 5 to 272 : 4000 to 20000.

Further, according to the fourth embodiment of the present invention, there is provided a method for producing a composition comprising an inclusion compound of isohumulones, comprising the steps of:
(a) mixing isohumulones, γCD and an aqueous solvent at a pH of 4.0 or below in a ratio of isohumulones : γCD : aqueous solvent = 1 : 5 to 33 : 20 to 627, where γCD : aqueous solvent = 1 : 4 to 19; and
(b) diluting the obtained composition with an aqueous solvent at a pH of 4.5 or below in a ratio of isohumulones : γCD : aqueous solvent = 1 : 5 to 272 : 4000 to 20000.

The obtained composition according to step (a) can comprise the isohumulones included in γCD in an amount of 70% or more relative to the total amount of the isohumulones.

In step (a), isohumulones, γCD and an aqueous solvent can be mixed at pH 9.0 or below.

In step (b), isohumulones, γCD and an aqueous solvent can be mixed at pH 4.5 or below, preferably at pH 3.5 or below. A pH after the dilution can be in a range of 2.0 to 4.5, preferably 2.0 to 3.5.

In step (b), a change of a membrane potential of the composition obtained in step (a) in an aqueous solvent can be 15% or less compared with that of a solution of the isohumulones alone.

In step (b), the concentrations of the isohumulones and the inclusion compound of isohumulones after the dilution can be in a range of 20 to 200 ppm.

According to the fourth embodiment of the present invention, there is further provided a method for producing a composition comprising an inclusion compound of isohumulones, comprising the steps of:
(c) mixing isohumulones, γCD and an aqueous solvent at a pH of 4.5 or below in a ratio of isohumulones : γCD : aqueous solvent = 1 : 5 to 272 : 20 to 1088, where γCD : aqueous solvent = 1 : 4 to 9; and
(d) diluting the obtained composition with an aqueous solvent at a pH of 4.5 or below in a ratio of isohumulones : γCD : aqueous solvent = 1 : 5 to 272 : 4000 to 20000.

The obtained composition according to step (c) can comprise the isohumulones included in γCD in an amount of 70% or more relative to the total amount of the isohumulones.

In step (c), isohumulones, γCD and an aqueous solvent can be preferably mixed at pH 3.5 or below. A pH during mixing can be in a range of 2.0 to 4.5, preferably pH 2.0 to 3.5.

According to the aforementioned method, the dilution can be carried out so that the pH value (X) after the dilution in step (d) satisfies (6.5-X)² x (γCD amount/isohumulone amount) > 50, where X ≤ 4.5.

In step (d), a pH after the dilution can be in a range of pH 4.5 or below, preferably pH 3.5 or below. A pH after the dilution can also be in a range of 2.0 to 4.5, preferably 2.0 to 3.5.

In step (d), a change of a membrane potential of the composition obtained in step (c) in an aqueous solution can be 15% or less compared with that of a solution of the isohumulones alone.

In step (d), the concentrations of isohumulones and the inclusion compound of isohumulones after the dilution can be preferably in a range of 20 to 200 ppm.

### Isohumulone

According to the present invention, isohumulones can be selected from the group consisting of isohumulone, isoadhumulone, isocohumulone and a combination thereof.

A commercially available isomerized hop extract can be favorably used as isohumulones. Isohumulones can also be produced according to a known method, for instance, can be synthesized according to a method described in "Chemistry and Analysis of Hop and Beer Bitter Acids", Developments in Food Science 27, M. Verzele, ELSEVIER. Isohumulones can be obtained by separating and purifying from a hop extract or an isomerized hop extract obtained by a method described hereinbelow.

An isomerized hop extract can be obtained by isomerizing an extract from lupulin grains of the hop (a hop extract). The hop is a perennial plant belonging to *Cannabaceae,* as well as a cone (a matured unfertilized female flower) thereof. The lupulin grains of the hop are raw materials for beer brewing and used for bittering and flavoring beer. In the brewing course of beer, humulones (humulone, cohumulone, adhumulone, posthumulone, prehumulone, *etc*.) in the hop are isomerized to isohumulones (isohumulone, isocohumulone, isoadhumulone, isoposthumulone, isoprehumulone, *etc*.) giving beer a characteristic taste and flavor.

A hop extract can be prepared by subjecting, for examples, cones or a compression thereof with or without crushing to an extraction process. Examples of extraction methods used in a beer brewery for preparing a hop extract include: an extraction method with ethanol solvent and an extraction method with supercritical carbon dioxide. The extraction method with supercritical carbon dioxide is characterized by a low content of polyphenols and highly concentrated bitter matters and essential oil components. Other general methods can also be applied to hop extraction, examples are: dipping hop cones or the crushed chips thereof in a solvent at a low or elevated temperature; extraction under agitation at an elevated temperature followed by filtration to obtain an extract; and percolation. The obtained extract is used as it is, after removing a solid matter by filtration or centrifugation at need, or after removing a solvent for partial concentration or drying, depending on a mode of the use.
After concentration or drying, the extract may be washed with a non-solubilizing solvent for purification and used, or dissolved or suspended in an appropriate solvent and used. According to the present invention, the solvent extract obtained as above may be dried according to a general method, such as vacuum drying or freeze drying, to a dried form of a hop extract and used.

Examples of a solvent for the above extraction include: water; C1 to C4 lower alcohols such as methanol, ethanol, propanol and butanol; lower alkyl esters such as ethyl acetate; glycols such as ethylene glycol, butylene glycol, propylene glycol and glycerin; other polar solvents such as ethyl ether, acetone and acetic acid; hydrocarbons such as benzene and hexane; known organic solvents of non-polar solvents such as ethyl ether and petroleum ether. Two or more of the above solvents may be used together.

Thereafter the extract may be filtered to remove insoluble matters, concentrated by vacuum, etc. and eventually removing a solvent to dryness according to the need. Extraction of cones or the crushed chips thereof by supercritical carbon dioxide or liquefied carbon dioxide is also preferable.

The crude extract contains humulones as well as their isomerized ones, isohumulones. The isohumulones to be used according to the present invention may be separated and purified from the crude extracts by applying a conventional method. Since isolated and purified isohumulones are hardly available in the market, the isomerized hop extract may be used as it is.

In order to increase the content of isohumulones, the crude extract should be preferably heated up in the presence of alkali or magnesium oxide for further isomerization. By the isomerization, humulones in the hop extract are completely converted to isohumulones.

The particulars of the isomerization will be described by way of an example; the hop extract is dissolved in an alcoholic solvent such as ethanol to which weak alkaline water is added and the mixture is heated (at about 92 to 93°C) in the presence of the alkaline water with a reflux to isomerize the hop extract by heat and to obtain an isomerized hop extract. The obtained isomerized hop extract may be, at need, concentrated or purified according to a known method (*e*.*g*. filtration, concentration under a reduced pressure and freeze-drying). As weak alkaline water (*e*.*g*. pH 8.5 to 9.5) used in the above isomerizing treatment, commercially available drinking water, such as "alkaline ionized water" or other bottled water, should better be used from the standpoint of safety. Commercial drinking water is highly safe with ample consumption experience. Further, the above isomerizing treatment is in principle the same as the heat isomerizing reaction occurred at the wort boiling process during beer brewing, and therefore safe from the standpoint of food supply.

As mentioned above, the isomerized hop extract may be used directly according to the present invention for the production of the composition and the food, however it is more preferable to use a fraction containing the active ingredient at higher concentration.

Hop extracts and isomerized extracts according to various methods are commercially available as beer additives. Therefore such commercial hop extracts or isomerized hop extracts may be used as they are or after a further isomerizing treatment at need, according to the present invention. Examples of a commercially available usable isomerized hop extract include: a hop extract obtained by extracting mainly humulones and lupulones from crushed hop cones using supercritical carbon oxide (*e*.*g*. CO2 Pure Resin Extract (Hopsteiner)), an extract obtained by isomerizing an extract of crushed hop cones using carbon dioxide (*e*.*g*. Isomerized Kettle Extract (SS. Steiner), main components: isohumulone and lupulones), a water-soluble extract prepared by isomerizing an extract of crushed hop cones using carbon dioxide and then modifying it to a potassium salt in a form of low viscosity liquid (*e*.*g*. ISOHOPCO2N (English Hop Products), ISOHOPR (Botanix), main component: isohumulone).
Naturally, it is possible to concentrate a fraction that contains isohumulones at higher concentration than the abovementioned extracts, by the abovementioned methods or the like.

Further, tetrahydro-isohumulone and p-isohumulone, which are produced by an organic synthesis technique and used in some area as a substitute or a supplement for hops, can be used according to the present invention.

### γCD and solvent

According to the present invention, commercially available γCD can be used. Any product form of γCD such as powder, granule and syrup may be used.

It is preferable that the purity of γCD should be higher; however, since αCD, βCD or carbohydrates other than cyclic saccharides such as glucose or maltose do not suppress the effect of the present invention, such mixture can be also used. Further, γCD can be analogous compounds of γCD such as branched γCD or modified γCD.

In the present invention, an aqueous medium, especially water, is preferably used as a medium facilitating the contact between the isohumulone and the γCD; however, there are no particular restrictions insofar as the isohumulones and the γCD can be homogeneously dissolved or dispersed. Examples of aqueous mediums other than water include: a pH buffer solution and a solvent mixture of a polar organic solvent and water.

### Dry form of the inclusion compound of isohumulones

According to the present invention, there is provided a dry form of the inclusion compound of isohumulones obtained by drying the composition containing isohumulones according to the present invention.

The dry form can be obtained by subjecting the composition according to the present invention and a paste-like substance obtained therefrom to spray-drying or freeze-drying.

A water content in the dry form can be 10% or less, or preferably 8% or less.

The dry form of the inclusion compound of isohumulones can preferably be a freeze-dried form, more preferably a freeze-dried powder form, according to a freeze-dry method.

The obtained dry form of the inclusion compound of isohumulones can be dissolved in an aqueous medium such as water to obtain a composition comprising the inclusion compound of isohumulones. In this case, an aqueous solvent (*e*.*g*. water and various drinks) with a pH of 5.5 or below, more preferably with a pH 4.5 or below is used for dissolution, in order to maintain the inclusion of isohumulones after the dissolution. A pH after the dissolution may be in a range of 3.5 to 5.5, preferably in a range of 4.5 to 5.5. Concentrations of isohumulones and the inclusion compound of isohumulones after the dissolution can be made preferably in a range of 20 to 200 ppm.

The dry form of the inclusion compound of isohumulones can be processed to a capsule, a tablet, etc. according to a known method. The dry form of the inclusion compound of isohumulones has excellent stability (Example 3). Therefore, high stability of the obtained capsule and tablet can be expected.

### Food

The inclusion compound of isohumulones according to the present invention releases isohumulones by treatment with an appropriate amount of α-amylase derived from swine pancreas (Sigma Aldrich, Inc.) to degrade γCD. This phenomenon can be recognized by clarification of a turbid solution with relatively low concentration of isohumulones by the above treatment, and glucose, maltose and maltotriose can be detected by analying the clarified solution. The above strongly indicates that γCD forming an inclusion compound is degraded by α-amylase to glucose, maltose and maltotriose, thereby releasing isohumulones.
Consequently, it can be deduced that when a food utilizing the inclusion compound of isohumulones according to the present invention is taken by humans, isohumulones are dissociated from γCD at the upper small intestine and absorbed at the small intestine rapidly without inhibition of the aforementioned beneficial physiological effects recognized on isohumulones.

The food according to the present invention is that containing an effective amount of isohumulones, which form an inclusion compound. The expression "containing an effective amount" herein means that isohumulones are contained in such an amount that they can be ingested in the range described below when each of the foods is taken in an ordinary amount. The inclusion compound of isohumulones according to the present invention can be added in the food according to the present invention in a form of a composition or a dry form. More specifically, the food according to the present invention may be that prepared by using the composition or the dry form according to the present invention as it is for a food, that prepared by further adding various proteins, carbohydrates, fats, trace elements, vitamins, etc., that formed in a liquid, semi-liquid, solid or paste type, or that prepared by mixing the same to ordinary foods. There are no restrictions on a form of the food according to the present invention, insofar as it can be ingested by mammals.

The term "food" used in the present invention includes health foods (e.g. a food for specified health uses, a food with nutrient function claims, a nutraceutical product), functional foods, and foods for patients.

The health food may be in an ordinary form of a food or in a form of a nutraceutical product (for example, a supplement).

Examples of the form of a nutraceutical product include: a tablet, a capsule (soft capsule, hard capsule), powders, a stick and a jelly.

There are no particular restrictions on a food form. For example, it may include a drink.

The food according to the present invention may be provided as a food for specified health uses having functions of isohumulones desired by consumers, such as a lipid metabolism improving function (WO03/068205), a blood-pressure reducing function and a blood vessel flexibility improving function (WO2004/064818). The above-mentioned "food for specified health uses" means a food subject to a possible legal control from the standpoint of public health, if the same is produced or sold with labeling of such a function.

According to the present invention, there is provided a food containing an effective amount of the inclusion compound of isohumulones according to the present invention with labeling of the aforementioned functions. The labeling of the various functions may be put on any of a food itself, a container, a package, an instruction, an attached document and an advertisement.

Specific examples of target daily foods, to which the inclusion compound of isohumulones according to the present invention may be added/mixed, include, but not limited thereto: foods containing carbohydrate, such as rice products, noodles, breads and pastas; various confectioneries such as western-style confectioneries as cookies or cakes, Japanese-style confectioneries as buns with a filling and steamed adzuki-bean pastes, candies, chewing-gums, and chilled or ice confectioneries as yogurt or custard pudding; alcoholic drinks, such as whiskey, bourbon, spirits, liqueur, wine, fruit liquors, sake, Chinese liquors, Japanese spirits, beer, non-alcohol beer with an alcohol level of not more than 1%, low-malt beer, cocktail; non-alcoholic drinks such as fruit drink, vegetable drink, fruit and vegetable drink, soft drink, cow's milk, soybean milk, lactic drink, yogurt drink, coffee, cocoa, tea, energy drink, sports drink, mineral water; and processed foods (including delicacies), such as processed food with eggs, with seefood (squid, octopus, shellfish, eel, etc.) and with meat (including pluck such as liver).

According to a more preferable embodiment of the present invention, examples of the target foods for addition/mixture include: alcoholic drinks (*e*.*g*. beer, low-malt beer), and non-alcoholic drinks (*e*.*g*. soft drink, fruit drink, vegetable drink, fruit and vegetable drink, tea, lactic drink).

Examples of the fruits used for a fruit drink and a fruit and vegetable drink include: apples, oranges, grapes, bananas, pears and Japanese apricots. Examples of the vegetables used for a vegetable drink and a fruit and vegetable drink include: tomatoes, carrots, celery, cucumbers and watermelons.

The "tea" means a drink infusing leaves of a tea tree (a tea tree is an evergreen tree belonging to *Theaceae*), or leaves or grains of other plants than the tea tree, and includes all of fermented tea, half-fermented tea and non-fermented tea. Specific examples of the tea include: Japanese tea (*e*.*g*. green tea, barley tea), black tea, herb tea (*e*.*g*. jasmine tea), Chinese tea (*e*.*g*. green tea, oolong tea) and roasted tea.

The "lactic drink" means a drink using as main raw materials raw milk, milk, or a food produced therefrom, and includes a drink using processed milk, such as nutrient-enriched milk, flavored milk and sweetened hydrolyzed milk, as a raw material in addition to milk and the like.

In the case of production of the drink (including a health food and functional food in a form of a drink) to be provided according to the present invention, carbohydrates, flavors, fruit juice, food additives as used in recipes for ordinary drinks may be appropriately added. For production of the drink a conventional method known in the art may be referred to, for example to "Soft Drinks (revised new edition)" (Korin Publishing Co., Ltd.).

Since isohumulones, an active ingeredient of the composition according to the present invention, are contained in a hop extract that has been ingested by humans for many years as a food, they are low in toxicity and can be used safely for mammals (*e*.*g*. humans, mouse, rat, rabbit, dog, cat, cattle, horse, pig, and monkey). The amount of intake of the active ingredient according to the present invention can be determined depending on the ingesting subject, the age and body weight of the subject, symptoms, the time of administration, the formulation type, the route of administration, the combination with other medicines, *etc.* For example, when the inclusion compound of isohumulones according to the present invention is ingested as a food, the same can be so formulated that the ingested amount falls within the range of 20 mg to 150 mg, preferably 35 mg to 55 mg per day per adult.

### Examples

The present invention is further illustrated by the following examples that are not intended as a limitation of the invention.

In the Examples, an isomerized hop extract ("Iso-Extract 30%", S. S. Steiner, Inc. New York (USA)) was used as a raw material of isohumulones, and the weight of the contained isohumulone was described. For γCD, "Dexipearl γ-100" ((γCD content ≥ 98%) Ensuiko Sugar Refining Co., Ltd.) was used. The amounts of isohumulones and γCD were expressed as substantially bone-dry weight, and water was expressed by the *status quo* weight.

### [Example 1] Establishment of a measuring method for an inclusion compound of isohumulones

Citrate buffer solutions (1 mM) with stepwise different pH values in a range of pH 2 to 7 were prepared and γCD solutions were prepared using them (Dexipearl γ-100 1% solutions). The isomerized hop extract was added to the respective γCD solutions so that the amounts of isohumulones in them become 100 ppm respectively. The solutions were used as samples for measurements.

The solutions became turbid depending on the pH values, and by measuring by a spectrophotometer (Beckman Coulter, wave length 600 nm and 720 nm) the absorbance values of the respective samples were found to decrease depending on the pH values (Figure 1). From the results of a comparative sensory evaluation by 5 panelists for the samples of pH 2 and pH 7, the bitter taste of the pH 2 sample was 5 to 20% of the bitter taste of the pH 7 sample, and a close correlation between the turbidity (clouding) and the degree of mitigation of the bitter taste was indicated.

Then the respective samples were centrifuged (3000 g x 10 min) and the contents of isohumulones in the supernatants were measured by an HPLC analysis (JASCO Corp.). Assuming that approximate amounts of the turbid isohumulones are calculated by reducing the measured amounts from the total amounts of isohumulones, the ratios of the turbid isohumulones amounts to the total amounts were plotted in Figure 2, which showed a substantially similar curve to that in Figure 1.

From the above results it was confirmed that the amount of isohumulones participating in the turbidity could be determined by measuring the amount of isohumulones that are not precipitated by centrifugation, in case direct measurement of the turbidity was difficult, and that such determined turbid isohumulones amount was deemed as the amount of the isohumulones (clathrated isohumulones), which bitter taste was mitigated by an effect of γCD.

### [Example 2] Experimentation to determine a ratio among isohumulones, γCD and water

### (1) Experimentation with an aqueous solution of pH 9

Aqueous solutions of pH 9 containing 5, 10 and 20% of γCD were prepared, and an isomerized hop extract was directly added and mixed to them to establish the weight ratios among isohumulones, γCD and water in the following table.

**[Table 1]**

| | γCD=5% | | | | γCD=10% | | | | γCD=20% | | |
|---|---|---|---|---|---|---|---|---|---|---|---|
| | isohumulones | γCD | water | | isohumulones | γCD | water | | isohumulones | γCD | water |
| ① | 1 | 20 | 380 | ⑤ | 1 | 67 | 600 | ⑨ | 1 | 67 | 266 |
| ② | 1 | 12 | 228 | ⑥ | 1 | 20 | 180 | ⑩ | 1 | 20 | 80 |
| ③ | 1 | 8 | 152 | ⑦ | 1 | 8 | 72 | ⑪ | 1 | 8 | 32 |
| ④ | 1 | 5 | 95 | ⑧ | 1 | 5 | 45 | ⑫ | 1 | 5 | 20 |

Most of the above compositions generated significantly turbidity and turned to slurries, but some generated little turbidity. Since the turbidity could be deemed as "isohumulones, which bitter taste was mitigated by an effect of γCD", according to Example 1, the samples were centrifuged (3000 g x 10 min) and the isohumulones in the supernatants were measured to calculate turbid isohumulones rates. In Figure 3 the results were plotted, thereby the weight ratio of isohumulones to γCD was taken along the abscissa, and the turbid isohumulones rate was taken along the ordinate.

From the standpoint of efficiency in obtaining the inclusion compound of isohumulones, the turbid isohumulones rate of 70% or higher is preferable. By adding an auxiliary line at 70% as illustrated in Figure 3, 4 intersections of A, B, C and D were observed. The weight ratios among isohumulones, γCD and water at the 4 intersections were shown in Table 2.

**[Table 2]**

| | Isohumulones | γCD | Water |
|---|---|---|---|
| Intersection A | 1 | 25 | 100 |
| Intersection B | 1 | 12.5 | 112.5 |
| Intersection C | 1 | 6 | 54 |
| Intersection D | 1 | 5 | 20 |

With the 10% γCD aqueous solution, the turbid isohumulones rate, namely the rate of isohumulones forming an inclusion compound, exceeds the 70% line in a range between intersections B and C. With the 20% γCD aqueous solution, the isohumulones rate exceeds the 70% line in a range between intersections A and D.

As the results, the efficient range for obtaining the inclusion compound of isohumulones is isohumulones : γCD : water = 1 : 5 to 25 : 20 to 112.5, thereby γCD : water = 1 : 4 to 9. Further, a preferable embodiment where the turbid isohumulones rate is equal to or beyond 70% is in a range of isohumulones : γCD : water = 1 : 6 to 12.5 : 54 to 112.5, where γCD : water = 1 : 4 to 9.

As shown in Example 1, the acidic conditions are favorable for forming the inclusion compound, and therefore it is clear to those skilled in the art that the inclusion compound can be also formed in the above ratio range below pH 9.0.

Similarly by taking intersections E and H of the curve of 20% γCD with the 80% line, and intersections F and G with the 90% line, the turbid isohumulones rate equal to or beyond 80% is to be attained in a range of isohumulones : γCD : water = 1 : 6 to 20 : 24 to 80, where γCD : water = 1 : 4, and equal to or beyond 90% is in a range of isohumulones : γCD : water = 1 : 7 to 14 : 28 to 56, where γCD : water = 1 : 4.

### (2) Experimentation with an aqueous solution of pH 4

An aqueous solution of pH 4 containing 5% of γCD was prepared, and the isomerized hop extract was directly added and mixed to it to establish the weight ratios among isohumulones, γCD and water in the following table.

**[Table 3]**

| | isohumulones | γCD | water |
|---|---|---|---|
| ① | 1 | 68 | 1292 |
| ② | 1 | 36 | 684 |
| ③ | 1 | 20 | 380 |
| ④ | 1 | 12 | 228 |
| ⑤ | 1 | 8 | 152 |
| ⑥ | 1 | 6 | 114 |
| ⑦ | 1 | 5 | 95 |

Most of the above compositions generated significantly turbidity and turned to slurries. They were centrifuged (3000 g x 10 min) respectively as in Example 2 (1), and the isohumulones in the supernatants were measured to calculate the turbid isohumulones rate. In Figure 4, the results were plotted, thereby the weight ratio of isohumulones to γCD was taken along the abscissa, and the turbid isohumulone rate was taken along the ordinate.

By adding an auxiliary line at 70% as illustrated in Figure 4 as in Example 2 (1), 2 intersections I, J were observed. The weight ratios among isohumulone, γCD and water at the 2 intersections were shown in Table 4.

**[Table 4]**

| | Isohumulones | γCD | Water |
|---|---|---|---|
| Intersection I | 1 | 33 | 627 |
| Intersection J | 1 | 5 | 95 |

The above results show that the turbid isohumulones rate reaches 70% or higher in a range of isohumulones : γCD : water = 1 : 5 to 33 : 20 to 627, where γCD : water = 1 : 4 to 19. As shown in Example 2 (1), the inclusion compound is favorably formed at a higher γCD concentration, and it is therefore apparent to those of skilled in the art that the inclusion compound can be formed at the above ratio also at a γCD concentration of 5% or more.

### [Example 3] Stability test of the inclusion compound of isohumulones

To a 20% γCD solution (pH 3.5), the isomerized hop extract was added and mixed to obtain high concentration slurry containing 2.6% of isohumulones (in this case, isohumulones : γCD : water = 1 : 7.7 : 30.7). The high concentration slurry was centrifuged (3000 g x 10 min) to recover solid matter, which was washed by a 1 mM citrate buffer solution (pH 3.5) and centrifuged again to recover a past-like inclusion compound of isohumulones. The paste was subjected to freeze-drying (Sample A). On the other hand, the isomerized hop extract per se, the source of isohumulones, was subjected to freeze-drying to be solidified and crushed for mixing with γCD powders. The mixture was used as a comparative sample (Sample B). The freeze-drying was carried out by mild heating of a frozen sample under a reduced pressure.

Samples A and B were stored unclosed at a temperature of 45°C and a moisture of 75% for 1 week, the white colored appearance of sample A remained and no change whatever was observed (Figure 5 left). On the other hand the appearance of sample B turned yellowish, and, sensually, smelled like fermented soy beans, showing apparent changes from the original status (Figure 5 right). The contents of isohumulones in the both samples were measured. There was no significant change in sample A, but a sharp decrease was observed in sample B (Figure 6).

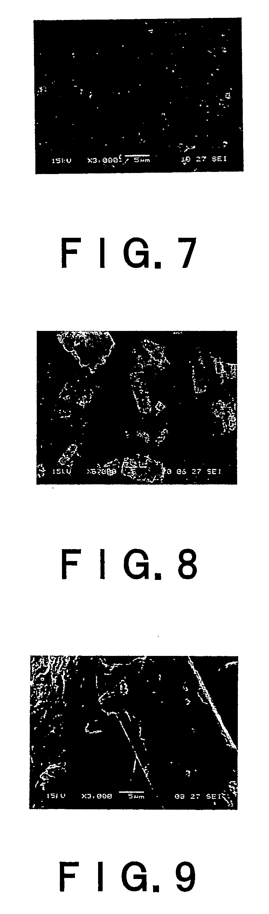

A histological comparison of samples A and B using an electron microscope to clarify the appearance differences was carried out. However, the observation of sample B was impossible, since it turned from a powder form to a resinous form quickly due to moisture absorption. The above also indicated that, although the components were same in the both samples, their properties were quite different. Compared, as a reference, with the powders of γCD, which were coarse irregular particles, sample A was confirmed to form clearly different columnar crystallites (Figure 7, Figure 8 and Figure 9).

The above results strongly indicate that sample A was clearly different from a plain mixture of isohumulones and γCD in properties and appearance and that sample A forms an inclusion compound.

### [Example 4] Structural analysis of an inclusion compound of isohumulones

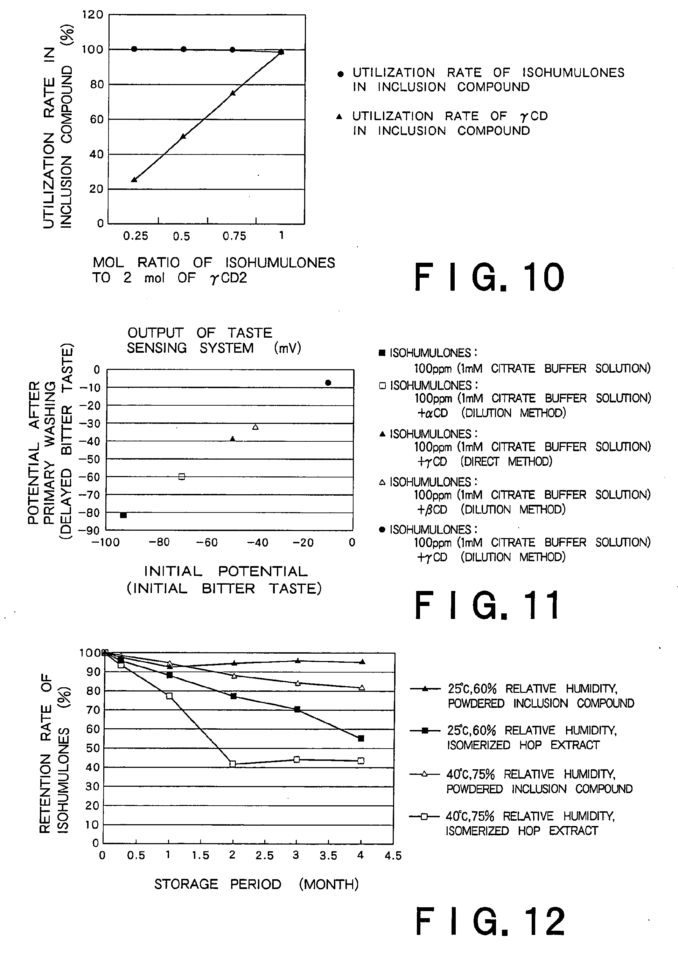

According to the method of Example 3, an inclusion compound of isohumulones was prepared 3 times and the content of isohumulones was measured by HPLC (JASCO Corp.) to be 11.9%, 12.2% and 11.9%. Assuming that 1 molecule of isohumulones and 2 molecules of γCD form an inclusion compound, the theoretical content of isohumulones is 12.2%. The results indicated that the inclusion compound of isohumulones was composed by the aforementioned molecule ratio. In accordance with the method of Example 3, an amount of isohumulones was changed stepwise from 0.25 mol to 1 mol based on 2 mol of γCD to prepare powders of the inclusion compound of isohumulones. The isohumulones contents in the obtained powders were measured by HPLC (HPLC) to obtain the results as shown in Figure 10. In a case where 2 mol of γCD was used for 1 mol of isohumulones, about 100% of both isohumulones and γCD were utilized in formation of the inclusion compound. This indicates that an inclusion compound of isohumulones was composed of 1 molecule of isohumulones and 2 molecules of γCD.

### Example 5] Comparison of a direct method and a dilution method in formation of an inclusion compound of isohumulones

### (1) Experimentation with a 20% γCD solution

To a 20% γCD solution (pH 3.5 or pH 4.5), the isomerized hop extract was directly added and mixed to establish the ratios among isohumulones, γCD and water shown in Table 5 wherein the water ratios are indicated at the left side of the arrows. Acidic water (1 mM citrate buffer solution) was then added to adjust the pH values to 3.5, 4.5, 5.5 and 6.5 so that the final water mixture ratios are adjusted to the right side values of the arrows in Table 5 (dilution method). The turbid isohumulones rate of the above case was defined as a dilution method turbid rate (A), and a rate where a mixture having the final ratio was directly prepared without dilution (direct method) was defined as a direct method turbid rate (B). The large differences between the two rates mean that initial preparation of a higher concentration solution of the inclusion compound followed by dilution under acidic conditions is more advantageous to obtain a solution of the inclusion compound with mitigated bitter taste, than directly preparing the final solution of the inclusion compound. The evaluation results of the differences between the two rates were represented as: ≥50% ("⊚"), 50%> ≥20% ("○"), 20%> "x" (Table 5).

**[Table 5]**

| | | | | | | | | | |
|---|---|---|---|---|---|---|---|---|---|
| | A: Dilution method turbid rate (%) C: (6.5-pH value)² B: Direct method turbid rate (%) D: C × γCD relative amount | | | | | | | | |
| [pH 3.5] (After dilution) | Isohumulones | γCD | water | A | B | A-B | Evaluation | C | D |
| | 1 | 136 | 544⇒8160 | 89.1 | 30.2 | 58.9 | ⊚ | 9 | 1224 |
| | 1 | 272 | 1088⇒8190 | 88.8 | 44.3 | 44.5 | ○ | 9 | 2448 |
| | Before dilution: pH 3.5 | | | | | | | | |
| [pH 4.5] (After dilution) | Isohumulones | γCD | water | A | B | A-B | Evaluation | C | D |
| | 1 | 34 | 136⇒8160 | 32.9 | 0 | 32.9 | ○ | 4 | 136 |
| | 1 | 68 | 272⇒8160 | 64.7 | 0 | 64.7 | ⊚ | 4 | 272 |
| | 1 | 136 | 544⇒8190 | 61.1 | 0 | 61.1 | ⊚ | 4 | 544 |
| | 1 | 272 | 1088⇒8190 | 57.7 | 0 | 57.7 | ⊚ | 4 | 1088 |
| | Before dilution: pH 4.5 | | | | | | | | |
| [pH 5.5] (After dilution) | Isohumulones | γCD | water | A | B | A-B | Evaluation | C | D |
| | 1 | 34 | 136⇒8160 | 3.7 | 0 | 3.7 | × | 1 | 34 |
| | 1 | 68 | 272⇒8160 | 38.7 | 0 | 38.7 | ○ | 1 | 68 |
| | 1 | 136 | 544⇒8190 | 43.2 | 0 | 43.2 | ○ | 1 | 136 |
| | 1 | 272 | 1088⇒8190 | 43.2 | 0 | 43.2 | ○ | 1 | 272 |
| | Before dilution: pH 4.5 | | | | | | | | |
| [pH 6.5] (After dilution) | Isohumulones | γCD | water | A | B | A-B | Evaluation | C | D |
| | 1 | 68 | 272⇒8160 | 0 | 0 | 0 | × | 0 | 0 |
| | 1 | 136 | 544⇒8190 | 0.3 | 0 | 0.3 | × | 0 | 0 |
| | 1 | 272 | 1088⇒8190 | 13.5 | 0 | 13.5 | × | 0 | 0 |
| | Before dilution: pH 4.5 | | | | | | | | |

It was concluded from the mixture ratios having the evaluation results of "○" or "⊚" that it is advantageous for prparation of an inclusion compound of isohumulones that a composition of isohumulones : γCD : water = 1 : 5 to 272 : 20 to 1088 should be prepared, and diluted at a pH of less than 6.5 in a ratio of isohumulones : γCD : water = 1 : 5 to 272 : 4000 to 20000. In that case, the dilution should be preferably carried out under the condition of the pH value (X) satisfying (6.5 - pH value)² x γCD relative amount > 50, and X < 6.5.

### (2) Experimentation with a 10% γCD solution

To a 10% γCD solution (pH 3.5 or pH 4.5), the isomerized hop extract was directly added and mixed to establish the mixture ratios among isohumulones, γCD and water shown in Table 6 wherein the water ratios are indicated at the left side of the arrows. Acidic water (1 mM citrate buffer solution) was then added to adjust the pH values to 3.5, 4.5 and 5.5 so that the final water mixture ratios are adfusted to the right side values of the arrows in Table 6 (dilution method). As in Example 5(1), the turbid isohumulones rate of the above case was defined as a dilution method turbid rate (A), and a rate where a mixture having the final ratio was directly prepared without dilution (direct method) was defined as a direct method turbid rate (B).

**[Table 6]**

| | | | | | | | | | |
|---|---|---|---|---|---|---|---|---|---|
| A: Dilution method turbid rate (%) C: (6.5-pH value)² B: Direct method turbid rate (%) D: C × γCD relative amount | | | | | | | | | |
| [PH3.5] (After dilution) | | | | | | | | | |
| Isohumulones | γCD | water | Turbid rate before dilution | A | B | A-B | Evaluation | C | D |
| 1 | 34 | 306⇒8190 | 99.3 | 73.4 | 11.7 | 61.7 | ⊚ | 9 | 306 |
| 1 | 68 | 612⇒8190 | 97.8 | 79.4 | 21.3 | 58.1 | ⊚ | 9 | 612 |
| 1 | 136 | 1224⇒8190 | 98.8 | 85.1 | 28.4 | 56.7 | ⊚ | 9 | 1224 |
| Before dilution: pH 3.5 | | | | | | | | | |
| [pH 4.5] (After dilution) | | | | | | | | | |
| Isohumulones | γCD | water | Turbid rate before dilution | A | B | A-B | Evaluation | C | D |
| 1 | 34 | 306⇒8190 | 95.3 | 20.3 | 0 | 20.3 | ○ | 4 | 136 |
| 1 | 68 | 612⇒8190 | 90.8 | 40.8 | 0 | 40.8 | ○ | 4 | 272 |
| 1 | 136 | 1224⇒8190 | 77.9 | 43.1 | 0 | 43.1 | ○ | 4 | 544 |
| Before dilution: pH 4.5 | | | | | | | | | |
| [pH 5.5] (After dilution) | | | | | | | | | |
| Isohumulones | γCD | water | Turbid rate before dilution | A | B | A-B | Evaluation | C | D |
| 1 | 34 | 306⇒8190 | 95.3 | 9.3 | 0 | 9.3 | × | 1 | 34 |
| 1 | 68 | 612⇒8190 | 90.8 | 24.7 | 0 | 24.7 | ○ | 1 | 68 |
| 1 | 136 | 1224⇒8190 | 77.9 | 20.1 | 0 | 20.1 | ○ | 1 | 136 |
| Before dilution: pH 5.5 | | | | | | | | | |

Concerning the results of Example 5(1) and Example 5(2), it is especially preferable that the turbid isohumulones rate is 70% or higher from the standpoint of efficiently obtaining the inclusion compound of isohumulones. Therefore, it is especially preferable that isohumulones, γCD and an aqueous solvent is diluted at a pH of 3.5 or below in a ratio of isohumulones : γCD : water = 1 : 5 to 272 : 4000 to 20000.

### [Example 6] Evaluation test of mitigation of the bitter taste of the inclusion compound of isohumulones

The degree of mitigation of the bitter taste of the inclusion compound of isohumulones was confirmed by sensory evaluation. The sensory evaluation was carried out with samples which were prepared by adding respectively the inclusion compound of isohumulones and the isomerized hop extract obtained in Example 2 to respective products (pH 3.6 to 4.3) so that the concentration of isohumulones is 50 ppm. The evaluation method was carried out in such a way that the isohumulones standard solutions having stepwise-predefined concentrations of 10, 20, 30, 40, 50 and 60 ppm were previously prepared and that 6 to 7 in-house panelists compare the samples and the standard solutions to find which concentration (bitter taste) of the standarad solutions the samples correspond to.

The results clearly showed the suppressive effect on the bitter taste of the inclusion compound of isohumulones (Table 7).

**[Table 7]**

| Trade name | Evaluation results ave±σ(ppm) | | t-Test results (significance level %) | pH of product | Number of panelists |
|---|---|---|---|---|---|
| | Composition | Hop extract | | | |
| Kirin Lemon | 7.2±3.7 | 34.7±5.2 | <0.001% | 3.6 | 6 |
| Amino Supli | 14.0±6.6 | 36.1±8.1 | <0.1% | 3.8 | 7 |
| Orange Juice | 4.2±4.1 | 29.0±2.4 | <0.001% | 3.9 | 6 |
| Vegetable juice | 8.2±8.3 | 41.3±4.5 | <0.01% | 4.0 | 6 |
| Kirin Lager | 38.1±4.7 | 55.7±7.3 | <0.1% | 4.3 | 7 |

### [Example 7] Confirmation test of mitigation of the bitter taste of the inclusion compound of isohumulones by membrane potential change

The degree of mitigation of the bitter taste of the composition comprising the inclusion compound of isohumulones was confirmed by measuring the change rate of the membrane potential. To the standard solution containing 100 ppm of isohumulones, various types of CD were added by the direct method or the dilution method (see Example 5) to prepare the samples shown in Table 8 and the solutions of isohumulones alone of the same concentrations and pH values were used as the control solutions. The membrane potential change was measured by a Taste Sensing System (Model SA402B, Probe used: SB2C00, Intelligent Sensor Technology, Inc.), and the ratio to the membrane potential change of the control solution was determined (membrane potential change rate). Additionally, a sensory evaluation similar to Example 6 was carried out concerning the mitigation degree of the bitter taste of the respective isohumulones-γCD compositions in reference to the solutions of isohumulones alone of the same concentrations and pH values. At the sensory evaluation, in order to obtain precise results, the samples were diluted 2-fold and 1% of glucose corresponding to the sweetness of 2.5% of CD was added to the standard solutions of isohumulones.

**[Table 8]**

| Sample | Initial potential | Potential change rate | Sensory evaluation of diluted solutions | Results of t-test with the standard solution |
|---|---|---|---|---|
| | (mV) | (%) | ave ± σ (ppm) | t-Test results |
| Standard solution of isohumulones | -93.02 | 100% | 45.0±6.4 | |
| + αCD 2.5% (Dilution method) | -69.69 | 74.9 | 38.1±9.2 | <5% |
| + βCD 2.5% (Dilution method) | -39.93 | 42.9 | | |
| + γCD 2.5% (Direct method) | -49.30 | 53.0 | 25.6±4.8 | <1% |
| + γCD 2.5% (Dilution method) | -9.95 | 10.5 | | |

| | | | | |
|---|---|---|---|---|
| Isohumulones: 100 ppm (1 mM citrate buffer solution) | | | | |

As a result, it was confirmed that there is a correlation between the membrane potential change rate and the mitigation effect of the bitter taste (Table 8). When a composition with the aimed concentration of isohumulones and γCD was prepared by the dilution method, the membrane potential change rate was confirmed to fall to 10.5%. Futher, the initial potential (mV) corresponding to the initial bitter taste, and the potential after a primary washing (mV) corresponding to the delayed bitter taste, of a composition of isohumulones and γCD prepared by the dilution method were confirmed to be significantly lower than those of the controls. (Figure 11)

### [Example 8] Test for storage stability of the inclusion compound of isohumulones

### (1) Test for storage stability of the freeze-dried powder

The powdered inclusion compound of isohumulones obtained in Example 3 was tested for storage stability. The storage stability test was carried out by measuring the retention rate of isohumulones over time. The retention rate of isohumulones was measured by HPLC (JASCO Corp.). The results confirmed that the retention rate of isohumulones of the powdered inclusion compound of isohumulones was maintained better than that of the powdered isomerized hop extract (Figure 12).

### (2) Accelerated long term test for storage stability of the spray-dried material

The high concentration slurry containing 2.6% of isohumulones obtained by adding and mixing an isomerized hop extract to a 20% γCD solution (pH 4.0) similarly as in Example 3 was subjected to spray-drying at various temperature conditions to obtain similar powdered inclusion compounds of isohumulones as by freeze-drying. The temperature conditions (incoming gas temperature / outgoing gas temperature) of spray-drying were: (1) 150°C / 80°C, (2) 180°C / 80°C, (3) 150°C / 70°C and (4) 150°C / 60°C. The storage stability of powdered inclusion compounds of isohumulones was tested after changing the moisture content in the powders by the following two treatments; Treatment (A): stored hermetically immediately after spray-drying, and (B): exposed to 60% relative humidity at 25°C for 1 week and then stored hermetically.

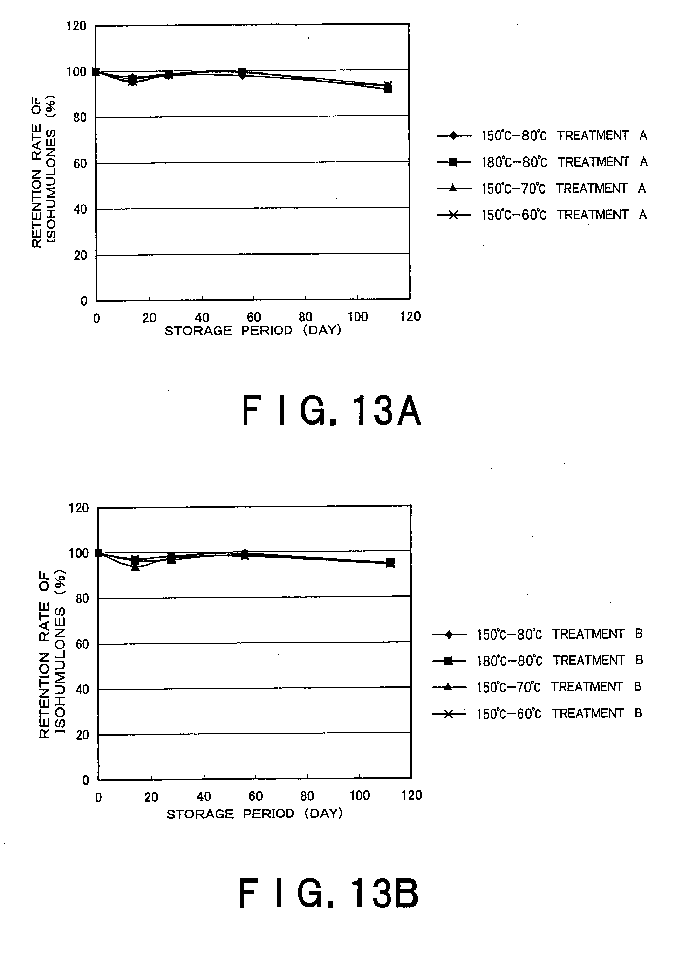

The storage stability test was carried out by measuring the retention rate of isohumulones over time. The retention rate of isohumulones was measured by HPLC (JASCO Corp.). As a result, it was confirmed that the retention rate of isohumulones of the powdered inclusion compound of isohumulones after a long term storage was well maintained irrespective of the spray-drying temperature conditions or the powder moisture content (Figures 13A and B), similarly as in the case of the powdered inclusion compound of isohumulones obtained by freeze-drying.

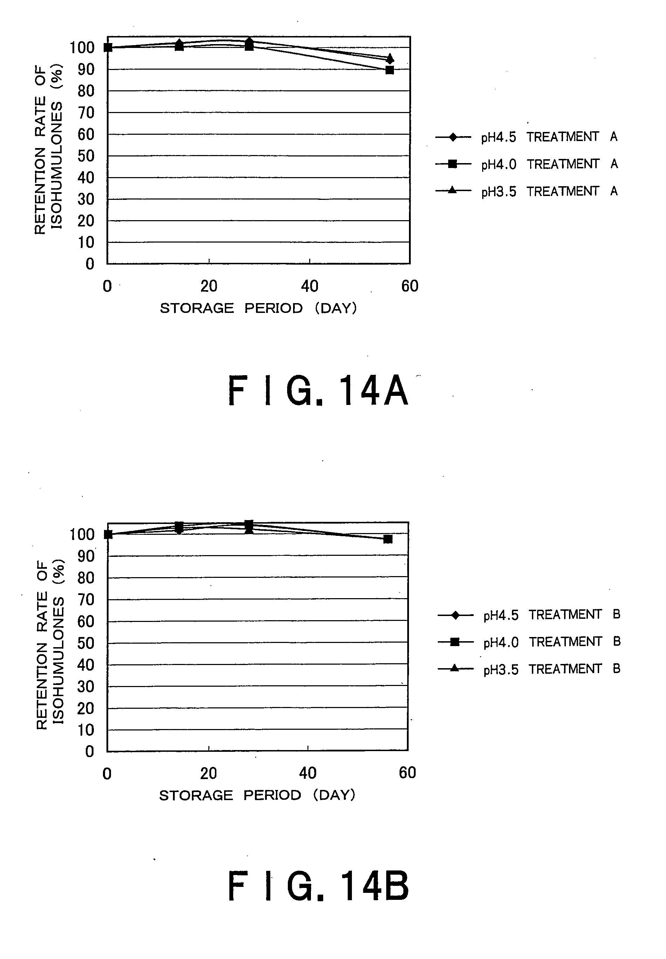

The 20% γCD solution was then adjusted to the pH of 3.5, 4.0 and 4.5, and the isomerized hop extract was added to the solution and mixed to obtain the high concentration slurries containing 2.6% of isohumulones. The slurries were subjected to spray-drying to obtain similar powdered inclusion compounds of isohumulones as by freeze-drying. The temperature condition (incoming gas temperature / outgoing gas temperature) of the spray-drying was 150°C / 60°C. The storage stability of the powdered inclusion compounds of isohumulones were tested after changing the moisture content in the powders by the following two treatments; Treatment (A): stored hermetically immediately after spray-drying, and (B): exposed to 60% relative humidity at 25°C for 1 week and then stored hermetically. As a result, it was confirmed that the retention rate of isohumulones of the powdered inlcusion compound of isohumulones obtained by spray-drying treatment after a long term storage was well maintained irrespective of the pH value of the high concentration slurry or the powder moisture content (Figures 14A and B), similarly as in the case of the powdered inclusion compound of isohumulones obtained by freeze-drying.

### (3) Accelerated long term test for storage stability of tablets and coated tablets

The obtained powdered inclusion compound of isohumulones in Example 8 (2) was tableted. The tableting conditions were as described below.

(1) Form: round 8^{φ}
(2) Tablet weight: uncoated tablet 250 mg, coating 50 mg, coated tablet 300 mg
(3) Coating material: About 20% of YeastWrap^{TN} used as an overcoat

| Raw materials | Content (%) | Content (g) |
|---|---|---|
| γCD Powdered inclusion compound | 50.70 | 152.1 |
| Excipient (lactose, for direct tableting granule lactose) | 45.10 | 135.3 |
| Silicon dioxide | 0.20 | 0.6 |
| Sugar ester | 2.00 | 6.0 |
| Vegetable oil | 2.00 | 6.0 |

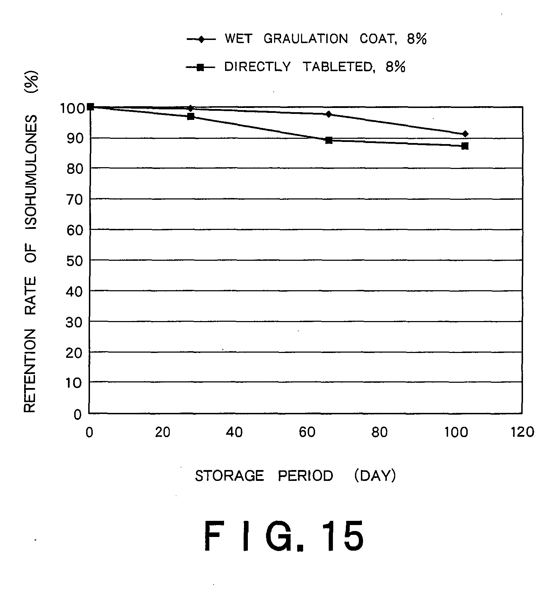

The above formulation was directly tableted to a sample (direct tablet) or was tableted to a sample after wet pelletizing (wet pellet tablet). Addition of lactose to the powdered inclusion compound improved the handling properties by suppressing the dispersion of the powdered inclusion compound of isohumulones. The tabletability was quite well for both the direct tablet and the wet pellet tablet, and according to the measurement of the isohumulones content in the tablets, the decrease of isohumulones during pelletizing and tableting was not observed. Next, the stability of the powdered inclusion compound of isohumulones in the direct tablet and the wet pellet tablet was tested. As a result, even after a long term storage, the retention rate of isohumulones in the tablet was maintained (Figure 15).

The above-described results showed that the storage stability of isohumulones was remarkably increased by forming the inclusion compound.

### [Example 9] Formulation examples of the inclusion compound of isohumulones

A soft capsule, a jelly food, a biscuit and a carbonated drink containing the inclusion compound of isohumulones (Example 3) were prepared according to the following recipes. In addition, the recipes were based on the premise that 36 mg of iso-α-acids was ingested daily, which was equivalent to a plan of daily ingestion of 324 mg of the inclusion compound of isohumulones.

### Soft capsule (1 capsule)

Powdered inclusion compound:81 mg (27 parts by weight)
Safflower oil:190 mg (63 parts by weight)
Beeswax:29 mg (10 parts by weight)

### (Intake per day 4 capsules)

### Jelly food (gelatin)

Powdered inclusion compound:324 mg (0.5 parts by weight)
Gelatin:3 g (2.5 parts by weight)
Water:97 g (80 parts by weight)
Sugar: 20 g (17 parts by weight)

### Jelly food (Kanten-agar)

Powdered inclusion compound:324 mg (0.3 parts by weight)
Kanten-agar:1 g (0.9 parts by weight)
Water: 99 g (90 parts by weight)
Sugar: 10 g (8.8 parts by weight)

### Biscuit

Powdered inclusion compound:324 mg (0.4 parts by weight)
Cake flour:30 g (31.1 parts by weight)
Corn starch:9 g (9.3 parts by weight)
Whole egg:15 g (15.6 parts by weight)
Sugar: 14 g (14.5 parts by weight)
Butter: 22 g (22.9 parts by weight)
Baking powder:0.2 g (0.2 parts by weight)
Water: 5.8 g (6 parts by weight)

### Carbonated drink

Powdered inclusion compound:324 mg (0.3 parts by weight)
Carbonated water: 100 g (99.7 parts by weight)

### [Example 10] Formulation examples of the inclusion compound of isohumulones (2)

A mixture of predetermined quantities of skim milk and sucrose was inoculated with *Lactobacillus paracasei,* and kept at 30 to 33°C for 12 to 24 hours for fermentation. The fermented liquid was diluted with water, to which the powdered inclusion compound of isohumulones (Example 3) was added to complete a lactic acid bacteria drink of a drinking yogurt type (lactic acid bacteria drink 1). Alternatively, the fermented liquid was diluted with water and orange juice, to which the powdered inclusion compound of isohumulones (Example 3) was added to complete a lactic acid bacteria drink of a juice type (lactic acid bacteria drink 2). Their recipes are shown in Table 9.

| | Lactic acid bacteria drink 1 | Lactic acid bacteria drink 2 |
|---|---|---|
| Powdered inclusion compound | 324mg | 324mg |
| Skim milk | 40g | 5g |
| Sucrose | 14g | 14g |
| Water | 45g | 45g |
| Fruit juice | 0g | 35g |

## Claims

1. An inclusion compound of isohumulones which is obtainable by mixing isohumulones, γ-cyclodextrin (γCD) and an aqueous solvent in a ratio of isohumulones : γCD : aqueous solvent = 1 : 5 to 25 : 20 to 112.5, where γCD : aqueous solvent = 1 : 4 to 9.

2. The inclusion compound according to claim 1, which is obtainable by mixing isohumulones, γ-cyclodextrin (γCD) and an aqueous solvent in a ratio of isohumulones : γCD : aqueous solvent = 1 : 6 to 12.5 : 54 to 112.5, where γCD : aqueous solvent = 1 : 4 to 9.

3. An inclusion compound of isohumulones which is obtainable by mixing isohumulones, γ-cyclodextrin (γCD) and an aqueous solvent at a pH of 4.0 or below in a ratio of isohumulones : γCD : aqueous solvent = 1 : 5 to 33 : 20 to 627, where γCD : aqueous solvent = 1 : 4 to 19.

4. An inclusion compound of isohumulones which is obtainable by mixing isohumulones, γ-cyclodextrin (γCD) and an aqueous solvent at a pH of 4.5 or below in a ratio of isohumulones : γCD : aqueous solvent = 1 : 5 to 272 : 20 to 1088, where γCD : aqueous solvent = 1 : 4 to 9.

5. The inclusion compound according to any one of claims 1 to 4, wherein the isohumulones are selected from the group consisting of isohumulone, isocohumulone, and isoadhumulone and a combination thereof.

6. The inclusion compound according to any one of claims 1 to 5, wherein the aqueous solvent is water.

7. A composition comprising an inclusion compound of isohumulones, which is obtainable by mixing isohumulones, γ-cyclodextrin (γCD) and an aqueous solvent in a ratio of isohumulones : γCD : aqueous solvent = 1 : 5 to 25 : 20 to 112.5, where γCD : aqueous solvent = 1 : 4 to 9.

8. The composition according to claim 7, which is obtainable by mixing isohumulones, γ-cyclodextrin (γCD) and an aqueous solvent in a ratio of isohumulones : γCD : aqueous solvent = 1 : 6 to 12.5 : 54 to 112.5, where γCD : aqueous solvent = 1 : 4 to 9.

9. A composition comprising an inclusion compound of isohumulones, which is obtainable by mixing isohumulones, γ-cyclodextrin (γCD) and an aqueous solvent at a pH of 4.0 or below in a ratio of isohumulones : γCD : aqueous solvent = 1 : 5 to 33 : 20 to 627, where γCD : aqueous solvent = 1 : 4 to 19.

10. The composition according to any one of claims 7 to 9, which comprises the isohumulones included in γCD in an amount of 70% or more relative to the total amount of the isohumulones.

11. The composition according to any one of claims 7 to 10, wherein a change of a membrane potential upon dissolution of the composition in an aqueous solvent is 15% or less compared with that of a solution of the isohumulones alone.

12. A composition comprising an inclusion compound of isohumulones, which is obtainable by mixing isohumulones, γ-cyclodextrin (γCD) and an aqueous solvent at a pH of 4.5 or below in a ratio of isohumulones : γCD : aqueous solvent = 1 : 5 to 272 : 20 to 1088, where γCD : aqueous solvent = 1 : 4 to 9.

13. The composition according to claim 12, which comprises the isohumulones included in γCD in an amount of 70% or more relative to the total amount of the isohumulones.

14. The composition according to claim 12 or 13, wherein a change of a membrane potential upon dissolution of the composition in an aqueous solvent is 15% or less compared with that of a solution of the isohumulones alone.

15. A composition comprising an inclusion compound of isohumulones, which is obtainable by diluting with an aqueous solvent the composition according to any one of claims 7 to 11 at a pH of 4.5 or below in a ratio of isohumulones : γCD : aqueous solvent = 1 : 5 to 272 : 4000 to 20000.

16. The composition according to claim 15, which is obtainable by diluting with an aqueous solvent the composition according to any one of claims 7 to 11 at a pH of 3.5 or below in a ratio of isohumulones : γCD : aqueous solvent = 1 : 5 to 272 : 4000 to 20000.

17. A composition comprising an inclusion compound of isohumulones, which is obtainable by diluting with an aqueous solvent the composition according to any one of claims 12 to 14 at a pH of 4.5 or below in a ratio of isohumulones : γCD : aqueous solvent = 1 : 5 to 272 : 4000 to 20000.

18. The composition according to claim 17, which is obtainable by diluting with an aqueous solvent the composition according to any one of claims 12 to 14 at a pH of 3.5 or below in a ratio of isohumulones : γCD : aqueous solvent = 1 : 5 to 272 : 4000 to 20000.

19. The composition according to claim 17 or 18, wherein a pH value X after the dilution satisfies (6.5-X)² × (γCD amount/isohumulone amount) > 50, where X ≤ 4.5.

20. The composition according to any one of claims 7 to 19, wherein the isohumulone are selected from the group consisting of isohumulone, isocohumulone, and isoadhumulone and a combination thereof.

21. The composition according to any one of claims 7 to 19, wherein the aqueous solvent is water.

22. A method for producing an inclusion compound of isohumulones or a composition comprising an inclusion compound of isohumulones, comprising mixing isohumulones, γ-cyclodextrin (γCD) and an aqueous solvent in a ratio of isohumulones : γCD : aqueous solvent = 1 : 5 to 25 : 20 to 112.5, where γCD : aqueous solvent = 1 : 4 to 9.

23. The method according to claim 22, comprising mixing isohumulones, γ-cyclodextrin (γCD) and an aqueous solvent in a ratio of isohumulones : γCD : aqueous solvent = 1 : 6 to 12.5 : 54 to 112.5, where γCD : aqueous solvent = 1 : 4 to 9.

24. A method of an inclusion compound of isohumulones or a composition comprising an inclusion compound of isohumulones, comprising mixing isohumulones, γ-cyclodextrin (γCD) and an aqueous solvent at a pH of 4.0 or below in a ratio of isohumulones : γCD : aqueous solvent = 1 : 5 to 33 : 20 to 627, where γCD : aqueous solvent = 1 : 4 to 19.

25. The method according to any one of claims 22 to 24, wherein the composition comprises the isohumulones included in γCD in an amount of 70% or more relative to the total amount of the isohumulones.

26. The method according to any one of claims 22 to 25, wherein a change of a membrane potential upon dissolution of the composition in an aqueous solvent is 15% or less compared with that of a solution of the isohumulones alone.

27. A method for producing an inclusion compound of isohumulones or a composition comprising an inclusion compound of isohumulones, comprising mixing isohumulones, γ-cyclodextrin (γCD) and an aqueous solvent at a pH of 4.5 or below in a ratio of isohumulones : γCD : aqueous solvent = 1 : 5 to 272 : 20 to 1088, where γCD : aqueous solvent = 1 : 4 to 9.

28. The method according to claim 27, wherein the composition comprises the isohumulones included in γCD in an amount of 70% or more relative to the total amount of the isohumulones.

29. The method according to claim 27 or 28, wherein a change of a membrane potential upon dissolution of the composition in an aqueous solvent is 15% or less compared with that of a solution of the isohumulones alone.

30. A method for producing a composition comprising an inclusion compound of isohumulones, comprising:
(a) mixing isohumulones, γ-cyclodextrin (γCD) and an aqueous solvent in a ratio of isohumulones : γCD : aqueous solvent = 1 : 5 to 25 : 20 to 112.5, where γCD : aqueous solvent = 1 : 4 to 9; and
(b) diluting the obtained composition with an aqueous solvent at a pH of 4.5 or below in a ratio of isohumulones : γCD : aqueous solvent = 1 : 5 to 272 : 4000 to 20000.

31. The method according to claim 30, which comprises:
(a) mixing isohumulones, γ-cyclodextrin (γCD) and an aqueous solvent in a ratio of isohumulones : γDD : aqueous solvent = 1 : 6 to 12.5 : 54 to 112.5, where γCD : aqueous solvent = 1 : 4 to 9; and
(b) diluting the obtained composition with an aqueous solvent at a pH of 4.5 or below in a ratio of isohumulones : γCD : aqueous solvent = 1 : 5 to 272 : 4000 to 20000.

32. A method for producing a composition comprising an inclusion compound of isohumulones, comprising:
(a) mixing isohumulones, γ-cyclodextrin (γCD) and an aqueous solvent at a pH of 4.0 or below in a ratio of isohumulones : γCD : aqueous solvent = 1 : 5 to 33 : 20 to 627, where γCD : aqueous solvent = 1 : 4 to 19; and
(b) diluting the obtained composition with an aqueous solvent at a pH of 4.5 or below in a ratio of isohumulones : γCD : aqueous solvent = 1 : 5 to 272 : 4000 to 20000.

33. A method for producing a composition comprising an inclusion compound of isohumulones, comprising:
(a) mixing isohumulones, γ-cyclodextrin (γCD) and an aqueous solvent at a pH of 4.5 or below in a ratio of isohumulones : γCD : aqueous solvent = 1 : 5 to 272 : 20 to 1088, where γCD : aqueous solvent = 1 : 4 to 9; and
(b) diluting the obtained composition with an aqueous solvent at a pH of 4.5 or below in a ratio of isohumulones : γCD : aqueous solvent = 1 : 5 to 272 : 4000 to 20000.

34. The method according to claim 33, wherein a pH value X after the dilution satisfies (6.5-X)² x (γCD amount/isohumulone amount) > 50, where X ≤ 4.5.

35. The method according to claim 33 or 34, wherein the concentrations of the isohumulones and the inclusion compound after the dilution are in a range of 20 to 200 ppm.

36. The composition according to any one of claims 7 to 21, which is in a form of a food.

37. The composition according to claim 36, wherein the food is a drink.

38. A dry form of the inclusion compound, which is obtainable by drying the composition according to any one of claims 7 to 21.

39. The dry form according to claim 38, wherein the drying is carried out by spray-drying or freeze-drying.

40. The dry form according to claim 38 or 39, wherein the water content is no more than 10%.

41. A food comprising the inclusion compound according to any one of claims 1 to 6, the composition according to claims 7 to 21, or the dry form according to any one of claims 38 to 40.

42. A composition comprising an inclusion compound of isohumulones, which can be produced by dissolving into an aqueous solvent the inclusion compound according to any one of claims 1 to 6, or the dry form of the inclusion compound according to any one of claims 38 to 40.

43. A capsule comprising the inclusion compound according to any one of claims 1 to 6, or the dry form according to any one of claims 38 to 40.

44. A tablet comprising the inclusion compound according to any one of claims 1 to 6, or the dry form according to any one of claims 38 to 40.
